# EUROPEAN PATENT SPECIFICATION

(11) **EP 1 752 447 B1**
(45) Date of publication and mention of the grant of the patent: **10.11.2010**
(21) Application number: 05017376.4
(22) Date of filing: 10.08.2005
(51) Int. Cl.: C07C 273/12, C07D 251/60

(54) **process for the integrated production of urea and melamine**
Verfahren zur integrierten Herstellung von Harnstoff und Melamin
Procédé de préparation integrée de l'urée et de la mélamine

(43) Date of publication of application: 14.02.2007
(73) Proprietor: UREA CASALE S.A., 6900 Lugano-Besso (CH)
(72) Inventor: Brunengo, Paolo, 6900 Lugano (CH)
(74) Representative: Zardi, Marco

(56) References cited:
- WO-A-98/08808
- WO-A-98/32731
- GB-A- 1 309 275

## Description

### Field of application

The present invention refers, in its most general aspect, to a process for the integrated production of urea and melamine synthesized from at least part of said urea.

In particular, the present invention refers to a process of the aforementioned type and comprising the steps of:
- carrying out a urea synthesis in a first urea synthesis reactor, fed with ammonia and carbon dioxide, obtaining a reaction mixture comprising urea and ammonium carbamate,
- carrying out melamine synthesis, with formation of off-gas comprising ammonia and carbon dioxide,
- carrying out a further urea synthesis in a second urea synthesis reactor, fed with said off-gas, with production of a respective reaction mixture comprising urea and ammonium carbamate,
- feeding said reaction mixture produced by said first reactor and said reaction mixture produced by said second reactor to a urea recovery unit, obtaining urea and an ammonium carbamate aqueous solution.

### Prior art

As it is known a well established process for the production of melamine from urea is the so-called integrated process, according to which the urea necessary for the aforementioned production is synthesized from ammonia and carbon dioxide in a urea production section of a so-called integrated plant, coexisting with and substantially flanked by a melamine synthesis section, included in the integrated plant itself.

Synthesis of urea from melamine synthesis waste gas is disclosed in GB-A-1309275.

In certain cases, the urea production section comprises a first urea synthesis reactor, a second urea synthesis reactor, and a urea recovery unit for recovering urea from the urea/ammonium carbamate aqueous mixtures produced in said reactors.

And it is known, for improved integration of such a process, that it is required to use, in the urea production section, the off-gases, comprising ammonia and carbon dioxide, which form in the melamine synthesis section, in order to produce a further amount of urea with them.

For such a purpose, said off-gases are fed to the second urea synthesis reactor, which operates at a pressure substantially equal to that of the melamine synthesis section. The aqueous reaction mixture thus obtained and comprising ammonia and carbon dioxide is subjected, in an appropriate decomposer, to a partial decomposition treatment of the ammonium carbamate contained in it and a recovery treatment of the free ammonia dissolved in it. The aqueous reaction mixture, comprising urea and ammonium carbamate, coming out from said first urea synthesis reactor, is also fed, for the same purpose, to the aforementioned decomposer. Moreover, the vapours leaving said decomposer are condensed, in an appropriate condenser, obtaining an ammonium carbamate aqueous solution, in turn recycled to said first synthesis reactor.

Although advantageous from the point of view of the use of the off-gases for the purposes of an increase in urea production, the prior art of integrated urea and melamine production and related plant actuated in the way schematically described above, has recognized drawbacks.

In particular, an overall yield of the urea production section is obtained that is unsatisfactory with respect to current market requirements, in particular in the case of high capacity plants.

### Summary of the invention

The technical problem underlying the present invention is that of devising and providing a process for the integrated production of urea and melamine of the type considered above, capable of overcoming the limitations and drawbacks cited with reference to the prior art, in a simple and effective manner, i.e. capable of totally satisfying the requirement of using the off-gases for the production of urea and at the same time capable of maximizing the yield in the urea production section.

This problem is solved, according to the present invention, by a process for the integrated production of urea and melamine synthesized from at least part of said urea, comprising the steps of:
- carrying out a urea synthesis in a first urea synthesis reactor, fed with ammonia and carbon dioxide, obtaining a reaction mixture comprising urea and ammonium carbamate,
- carrying out melamine synthesis, with formation of off-gas comprising ammonia and carbon dioxide,
- carrying out a further urea synthesis in a second urea synthesis reactor, fed with said off-gas, with production of a respective reaction mixture comprising urea and ammonium carbamate,
- feeding said reaction mixture produced by said first reactor and said reaction mixture produced by said second reactor to a urea recovery unit, obtaining urea and an ammonium carbamate aqueous solution,
said process is characterized by the fact of:
- feeding said ammonium carbamate aqueous solution obtained in said recovery unit to said second urea synthesis reactor.

Further characteristics and advantages of the process for the integrated production of urea and melamine according to the present invention shall become clear from the following description of a preferred embodiment thereof, made for indicating and not limiting purposes, with reference to the attached drawings.

### Brief description of the drawings

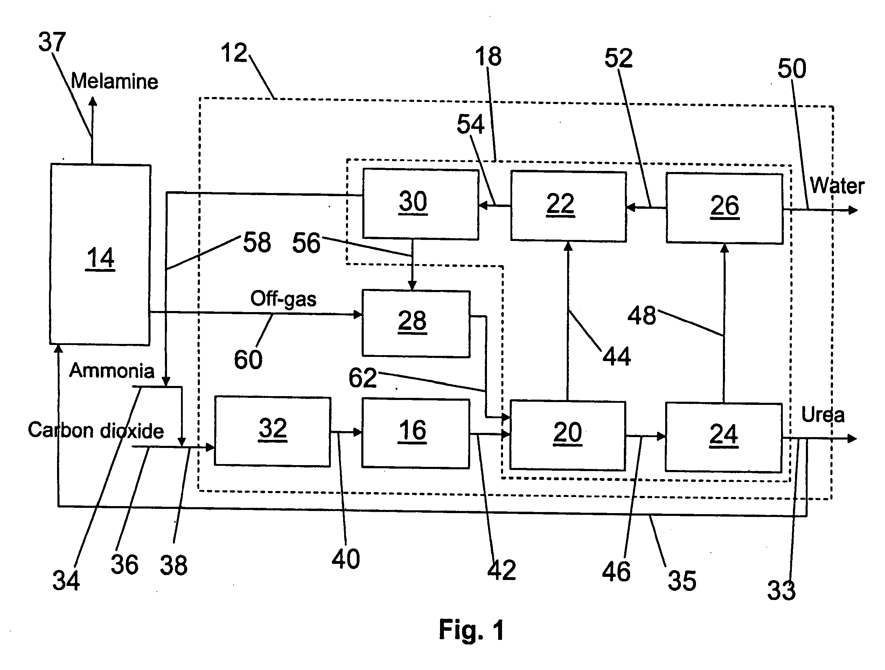

Figure 1 schematically represents a block diagram of a process for the integrated production of urea and melamine according to the present invention.

### Detailed description of a preferred embodiment

With reference to the figure, the process for the integrated production of urea and melamine according to the present invention is based upon urea synthesis, in a respective urea production section 12, starting from ammonia and carbon dioxide and upon melamine synthesis, in a respective melamine synthesis section 14, starting from at least a part of the urea produced in the urea production section 12.

In melamine synthesis, off-gases are formed comprising ammonia and carbon dioxide (and - in minimal quantities - steam) that are in turn used in urea synthesis.

In particular, said ammonia and said carbon dioxide are fed into a first urea synthesis reactor 16, which preferably operates at high pressure, i.e. a pressure approximately between 100 and 450 bar. A first reaction mixture comprising urea and ammonium carbamate is discharged from said first reactor 16 and is sent into a urea recovery unit 18.

Further urea is synthesized in a second urea synthesis reactor 28, included in the urea production section 12, into which said off-gases are fed. A second reaction mixture comprising urea and ammonium carbamate is discharged from said reactor 16 and sent into the urea recovery unit 18.

In the urea recovery unit 18, the urea contained in said first and said second reaction mixture is separated from an ammonium carbamate aqueous solution.

More specifically, in a decomposition section 20, comprising at least one decomposer, included in the urea recovery unit 18, the reaction mixtures undergo a partial decomposition treatment of the ammonium carbamate and a partial separation treatment of the free ammonia in aqueous solution present in said first mixture, obtaining an aqueous solution essentially comprising urea. The vapours leaving said decomposition section 20 are at least partially condensed in a condensation section 22, comprising at least one condenser, included in the urea recovery unit 18, obtaining a carbamate aqueous solution, which is recycled in the urea production section 12 in the way described hereafter.

The aqueous solution essentially comprising urea undergoes a finishing treatment in a suitable *per* se conventional urea finishing apparatus 24, which is arranged downstream of the decomposition section 20, obtaining urea ready for packaging and waste water. The Waste water is subjected to purification in a suitable *per* se conventional waste water treatment apparatus 26, which is arranged downstream of the urea finishing apparatus 24, before being released into the environment.

In the waste water treatment apparatus 26 vapours are generated that are sent to the condensation section 22, for their at least partial condensation.

In accordance with an aspect of the present invention, said ammonium carbamate aqueous solution is fed to the second urea synthesis reactor 28. The second reactor 28 preferably operates at high pressure, i.e. at a pressure approximately between 70 and 250 bar.

The second reaction mixture is treated in the decomposition section 20, together with the first reaction mixture obtained in the first urea synthesis reactor 16, so that both the urea contained in the first reaction mixture and the further urea contained in the second reaction mixture are separated in the unit 18 at the same time.

The off-gases produced in the melamine synthesis section 14, comprising ammonia and carbon dioxide, which are fed into the second urea synthesis reactor 28, react with the ammonium carbamate solution, advantageously supplying the heat necessary for the reaction in the second reactor 28 itself.

Preferably, said ammonium carbamate aqueous solution is fed to said second urea synthesis reactor 28 after a ammonia recovery carried out in a *per* se conventional ammonia recovery apparatus 30. The ammonia recovered here is then sent to the ammonia feed for the first reactor 16.

Preferably, the ammonia and carbon dioxide are mixed before entry into the first reactor 16, and enter into it in a suitable proportion, in a *per* se conventional manner.

Preferably, the aforementioned ammonia and carbon dioxide mixture, before being made to react in the first reactor 16, is subjected to condensation, preferably partial, in a further condenser 32, in order to recover heat and at the same time to suitably lower the temperature of the mixture to be fed into the first reactor 16, so as to control the reactor outlet temperature.

As far as the operating pressures are concerned, the decomposition section 20 preferably operates at medium pressure, for example at a pressure approximately between 10 and 80 bar. The first reaction mixture leaving the first reactor 16 is expanded at the operating pressure of the decomposition section 20, before being fed therein.

Advantageously, the second reactor 28 operates at a pressure similar to that of the melamine synthesis reactor included in said melamine synthesis section 14, so that the off-gases are fed to the second reactor 28 without having to operate any compression on them. The second reaction mixture leaving the second reactor 28 is expanded to the operating pressure of the decomposition section 20, before being fed therein.

The further condenser 32 preferably operates at the same pressure as the first reactor 16.

The present invention also refers to a plant for the integrated production of urea and melamine, which carries out the process indicated above.

The plant comprises the urea production section 12, wherein the urea is discharged with a duct 33, and the melamine synthesis section 14, wherein the melamine is discharged with a duct 37. The section 14 is fed with at least part of said urea through a duct 35 that extends from the duct 33.

The urea production section 12 is fed by a duct 34 carrying ammonia and by a duct 36 carrying carbon dioxide. The ducts 34 and 36 join into a duct 38 that feeds the first urea synthesis reactor 16.

In the preferred embodiment schematized in figure 1, the duct 38 feeds the further condenser 32 and a duct 40 is provided for the fluid communication between the further condenser 32 and the first urea synthesis reactor 16.

A duct 42 is provided for the fluid communication between the first reactor 16 and the decomposition section 20.

The decomposition section 20 is in communication with the condensation section 22 through a duct 44 and with the apparatus 24 through a duct 46.

A duct 48 is provided for the fluid communication between the apparatus 24 and the apparatus 26.

The apparatus discharges water through a duct 50 and is in communication with the condensation section 22 through a duct 52.

A duct 54 is provided for the fluid communication between the condensation section 22 and the apparatus 30.

The apparatus 30 is in communication with the second reactor 28 through a duct 56 and with the duct 34 through a duct 58. The duct 56 is used to completely recycle the carbamate aqueous solution generated in the urea recovery unit 18 to the second reactor 28. A duct 60 is provided for the fluid communication between the section 14 and the second reactor 28.

The second reactor 28 is in fluid communication with the decomposition section 20 through a duct 62. In an alternative embodiment the duct 62 can be fitted onto the duct 42 that is - as stated - in fluid communication with the decomposition section 20 itself.

From the previous description it can clearly be seen that a process for the integrated production of urea and melamine according to the invention achieves numerous advantages the first of which lies in the fact that an unusually high overall yield of the urea production section is obtained.

## Claims

1. Process for the integrated production of urea and melamine synthesized from at least part of said urea, comprising the steps of:
- carrying out a urea synthesis in a first urea synthesis reactor (16), fed with ammonia and carbon dioxide, obtaining a reaction mixture comprising urea and ammonium carbamate, .
- carrying out melamine synthesis, with formation of off-gas comprising ammonia and carbon dioxide,
- carrying out a further urea synthesis in a second urea synthesis reactor (28), fed with said off-gas, with production of a respective reaction mixture comprising urea and ammonium carbamate,
- feeding said reaction mixture produced by said first reactor (16) and said reaction mixture produced by said second reactor (28) to a urea recovery unit (18), obtaining urea and an ammonium carbamate aqueous solution, said process is **characterized by** the fact of:
- feeding said ammonium carbamate aqueous solution obtained in said recovery unit (18) to said second urea synthesis reactor (28).

2. Process according to claim 1, **characterized in that** the reaction mixture obtained in said first reactor (16) is subjected, in a decomposition section (20), to a partial decomposition treatment of the ammonium carbamate and a partial separation treatment of the free ammonia in aqueous solution present in said reaction mixture, obtaining an aqueous solution essentially comprising urea, the vapours leaving from said decomposition section (20) being at least partially condensed in a condensation section (22), obtaining said carbamate aqueous solution.

3. Process according to claim 2, **characterized in that** said aqueous solution essentially comprising urea undergoes a finishing treatment in a urea finishing apparatus (24), which is arranged downstream of the decomposition section (20), obtaining urea ready for packaging and waste water, which is subjected to purification in a waste water treatment apparatus (26) arranged downstream of the urea finishing apparatus (24) before being released into the environment.

4. Process according to claim 3, **characterized in that** vapours are generated in the waste water treatment apparatus (26) that are sent to said condensation section (22) for their at least partial condensation.

5. Process according to claim 2, **characterized in that** said reaction mixture obtained in said second urea synthesis reactor (28) is treated in the decomposition section (20), together with said reaction mixture obtained in the first urea synthesis reactor (16).

6. Process according to claim 1, **characterized in that** said ammonium carbamate aqueous solution is fed to said second urea synthesis reactor (28) after prior ammonia recovery, carried out in an ammonia recovery apparatus (30).

7. Process according to claim 6, **characterized in that** the ammonia, recovered in said ammonia recovery apparatus (30), is sent to the ammonia feed for the first reactor (16).

8. Process according to claim 1, **characterized in that** said ammonia and said carbon dioxide are mixed before entry into the first reactor (16).

9. Process according to claim 8, **characterized in that** the mixture of ammonia and carbon dioxide, before being made to react in the first reactor (16), is subjected to a partial condensation in a further condenser (32).

10. Plant for the integrated production of urea and melamine, comprising a urea production section (12) and a melamine synthesis section (14), wherein said urea production section (12) comprises a first urea synthesis reactor (16), a second urea synthesis reactor (28) and a urea recovery unit (18), ducts (42, 62) being provided between said first reactor (16) and said urea recovery unit (18) and between said second reactor (28) and said urea recovery unit (18), to discharge respective reaction mixtures produced in said first (16) and second reactor (28) into said urea recovery unit (18), a duct (60) being provided between said melamine synthesis section (14) and said second reactor (28), to feed the off-gases, comprising ammonia and carbon dioxide, generated in said melamine synthesis section (14), into said second reactor (28), a duct (56) being provided between said urea recovery unit (18) and said second reactor (28), to completely recycle an ammonium carbamate aqueous solution generated in said urea recovery unit (18) to said second reactor (28).

## Patentansprüche

1. Verfahren zur integrierten Herstellung von Harnstoff und Melamin, das aus zumindest einem Teil des Harnstoffs synthetisiert ist, umfassend die Schritte:
- Ausführen einer Harnstoffsynthese in einem ersten, mit Ammoniak und Kohlenstoffdioxid gespeisten Harnstoffsynthesereaktor (16) und Erhalten eines Reaktionsgemischs, umfassend Harnstoff und Ammoniumcarbamat,
- Ausführen einer Melaminsynthese mit Bildung von Abgas, umfassend Ammoniak und Kohlenstoffdioxid,
- Ausführen einer weiteren Harnstoffsynthese in einem zweiten, mit dem Abgas gespeisten Harnstoffsynthesereaktor (28), mit Herstellung eines entsprechenden Reaktionsgemischs, umfassend Harnstoff und Ammoniumcarbamat,
- Einspeisen des vom ersten Reaktor (16) hergestellten Reaktionsgemischs und des vom zweiten Reaktor (28) hergestellten Reaktionsgemischs in eine Harnstoffgewinnungseinheit (18) und Erhalten von Harnstoff und einer wässrigen Ammoniumcarbamatlösung,
wobei das Verfahren **dadurch gekennzeichnet ist**:
- Einspeisen der in der Gewinnungseinheit (18) erhaltenen wässrigen Ammoniumcarbamatlösung in den zweiten Harnstoffsynthesereaktor (28).

2. Verfahren gemäß Anspruch 1, **dadurch gekennzeichnet, dass** das im ersten Reaktor (16) erhaltene Reaktionsgemisch in einem Zersetzungsabschnitt (20) einer partiellen Zersetzungsbehandlung des Ammoniumcarbamats und einer partiellen Abtrennungsbehandlung des freien Ammoniaks in wässriger Lösung, der im Reaktionsgemisch vorhanden ist, unterworfen wird, wobei eine im Wesentlichen Harnstoff umfassende wässrige Lösung erhalten wird, wobei die, aus dem Zersetzungsabschnitt (20) austretenden Dämpfe zumindest teilweise in einem Kondensationsabschnitt (22) kondensiert werden, so dass die wässrige Carbamatlösung erhalten wird.

3. Verfahren gemäß Anspruch 2, **dadurch gekennzeichnet, dass** die im Wesentlichen Harnstoff umfassende wässrige Lösung in einer Harnstoff-Endbearbeitungsanlage (24), die dem Zersetzungsabschnitt (20) nachgeschaltet ist, einer abschließenden Behandlung unterzogen wird, wobei zum Verpacken fertiger Harnstoff und Abwasser erhalten wird, das in einer Abwasserbehandlungsanlage (26), die der Harnstoff-Endbearbeitungsanlage (24) nachgeschaltet ist, der Reinigung ausgesetzt wird, bevor es in die Umwelt entlassen wird.

4. Verfahren gemäß Anspruch 3, **dadurch gekennzeichnet, dass** Dämpfe in der Abwasserbehandlungsanlage (26) erzeugt werden, die für ihre zumindest teilweise Kondensation zum Kondensationsabschnitt (22) geleitet werden.

5. Verfahren gemäß Anspruch 2, **dadurch gekennzeichnet, dass** das im zweiten Harnstoffsynthesereaktor (28) erhaltene Reaktionsgemisch im Zersetzungsabschnitt (20) zusammen mit dem im ersten Harnstoffsynthesereaktor (16) erhaltenen Reaktionsgemisch behandelt wird.

6. Verfahren gemäß Anspruch 1, **dadurch gekennzeichnet, dass** die wässrige Ammoniumcarbamatlösung nach vorheriger Ammoniakgewinnung, die in einer Ammoniakgewinnungsanlage (30) ausgeführt wird, in den zweiten Harnstoffsynthesereaktor (28) eingespeist wird.

7. Verfahren gemäß Anspruch 6, **dadurch gekennzeichnet, dass** der in der Ammoniakgewinnungsanlage (30) gewonnene Ammoniak zu dem Ammoniak geführt wird, der in den ersten Reaktor (16) eingespeist wird.

8. Verfahren gemäß Anspruch 1, **dadurch gekennzeichnet, dass** der Ammoniak und das Kohlenstoffdioxid vor dem Eintritt in den ersten Reaktor (16) gemischt sind.

9. Verfahren gemäß Anspruch 8, **dadurch gekennzeichnet, dass** das Gemisch von Ammoniak und Kohlenstoffdioxid einer partiellen Kondensation in einem weiteren Kondensator (32) unterworfen wird, bevor es im ersten Reaktor (16) zur Reaktion gebracht wird.

10. Anlage für die integrierte Herstellung von Harnstoff und Melamin, umfassend einen Harnstoffherstellungsabschnitt (12) und einen Melaminsyntheseabschnitt (14), wobei der Harnstoffherstellungsabschnitt (12) einen ersten Harnstoffsynthesereaktor (16), einen zweiten Harnstoffsynthesereaktor (28) und eine Harnstoffgewinnungseinheit (18) umfasst, wobei Leitungen (42, 62) zwischen dem ersten Reaktor (16) und der Harnstoffgewinnungseinheit (18) und zwischen dem zweiten Reaktor (28) und der Harnstoffgewinnungseinheit (18) bereit gestellt sind, um die jeweiligen im ersten (16) und zweiten Reaktor (28) hergestellten Reaktionsgemische in die Harnstoffgewinnungseinheit (18) abzulassen, wobei eine Leitung (60) zwischen dem Melaminsyntheseabschnitt (14) und dem zweiten Reaktor (28) bereit gestellt ist, um die im Melaminsyntheseabschnitt (14) erzeugten Abgase, umfassend Ammoniak und Kohlenstoffdioxid, in den zweiten Reaktor (28) zu führen, wobei eine Leitung (56) zwischen der Harnstoffgewinnungseinheit (18) und dem zweiten Reaktor (28) bereit gestellt ist, um eine wässrige Ammoniumcarbamatlösung, die in der Harnstoffgewinnungseinheit (18) erzeugt wurde, in den zweiten Reaktor (28) zum vollständigen Recycling zu führen.

## Revendications

1. Procédé pour la production intégrée durée et de mélamine synthétisée à partir d'au moins une partie de ladite urée, comportant les étapes consistant à :
- exécuter une synthèse d'urée dans un premier réacteur de synthèse d'urée (16), alimenté en ammoniac et en dioxyde de carbone, en obtenant un mélange réactionnel comportant de l'urée et du carbamate d'ammonium,
- exécuter une synthèse de mélamine, avec formation d'effluents gazeux comportant de l'ammoniac et du dioxyde de carbone,
- exécuter une synthèse d'urée supplémentaire dans un second réacteur de synthèse d'urée (28), alimenté en dits effluents gazeux, avec production d'un mélange réactionnel respectif comportant de l'urée et du carbamate d'ammonium,
- transférer ledit mélange réactionnel produit par ledit premier réacteur (16) et ledit mélange réactionnel produit par ledit second réacteur (28) vers une unité de récupération d'urée (18), en obtenant de l'urée et une solution aqueuse de carbamate d'ammonium,
ledit procédé étant **caractérisé par** le fait de :
- transférer ladite solution aqueuse de carbamate d'ammonium obtenue dans ladite unité de récupération (18) vers ledit second réacteur de synthèse d'urée (28).

2. Procédé selon la revendication 1, **caractérisé en ce que** le mélange réactionnel obtenu dans ledit premier réacteur (16) est soumis, dans un tronçon de décomposition (20), à un traitement de décomposition partielle du carbamate d'ammonium et à un traitement de séparation partielle de l'ammoniac libre en solution aqueuse présent dans ledit mélange réactionnel, en obtenant une solution aqueuse comportant essentiellement de l'urée, les vapeurs quittant ledit tronçon de décomposition (20) étant au moins partiellement condensées dans un tronçon de condensation (22), en obtenant ladite solution aqueuse de carbamate.

3. Procédé selon la revendication 2, **caractérisé en ce que** ladite solution aqueuse comportant essentiellement de l'urée subit un traitement de finition dans un dispositif de finition d'urée (24), qui est agencé en aval du tronçon de décomposition (20), en obtenant de l'urée prête pour un conditionnement et des eaux usées, qui sont soumises à purification dans un dispositif de traitement des eaux usées (26) agencé en aval du dispositif de finition durée (24) avant d'être libérées dans l'environnement.

4. Procédé selon la revendication 3, **caractérisé en ce que** des vapeurs sont générées dans le dispositif de traitement des eaux usées (26) qui sont envoyées vers ledit tronçon de condensation (22) pour leur condensation au moins partielle.

5. Procédé selon la revendication 2, **caractérisé en ce que** ledit mélange réactionnel obtenu dans ledit second réacteur de synthèse d'urée (28) est traité dans le tronçon de décomposition (20), en association avec ledit mélange réactionnel obtenu dans le premier réacteur de synthèse d'urée (16).

6. Procédé selon la revendication 1, **caractérisé en ce que** ladite solution aqueuse de carbamate d'ammonium est transférée vers ledit second réacteur de synthèse d'urée (28) après récupération préalable d'ammoniac, exécutée dans un dispositif de récupération d'ammoniac (30).

7. Procédé selon la revendication 6, **caractérisé en ce que** l'ammoniac, récupéré dans ledit dispositif de récupération d'ammoniac (30), est envoyé vers l'alimentation en ammoniac du premier réacteur (16).

8. Procédé selon la revendication 1, **caractérisé en ce que** ledit ammoniac et ledit dioxyde de carbone sont mélangés avant une entrée dans le premier réacteur (16).

9. Procédé selon la revendication 8, **caractérisé en ce que** le mélange d'ammoniac et de dioxyde de carbone, avant d'être amené à réagir dans le premier réacteur (16), est soumis à une condensation partielle dans un condenseur supplémentaire (32).

10. Installation pour la production intégrée d'urée et de mélamine, comportant un tronçon de production durée (12) et un tronçon de synthèse de mélamine (14), dans laquelle ledit tronçon de production d'urée (12) comporte un premier réacteur de synthèse d'urée (16), un second réacteur de synthèse d'urée (28) et une unité de récupération d'urée (18), des conduites (42, 62) étant agencées entre ledit premier réacteur (16) et ladite unité de récupération d'urée (18) et entre ledit second réacteur (28) et ladite unité de récupération d'urée (18), pour décharger des mélanges réactionnels respectifs produits dans lesdits premier (16) et second (28) réacteurs dans ladite unité de récupération d'urée (18), une conduite (60) étant agencée entre ledit tronçon de synthèse de mélamine (14) et ledit second réacteur (28), pour transférer les effluents gazeux, comportant de l'ammoniac et du dioxyde de carbone, générés dans ledit tronçon de synthèse de mélamine (14), dans ledit second réacteur (28), une conduite (56) étant agencée entre ladite unité de récupération d'urée (18) et ledit second réacteur (28), pour recycler entièrement une solution aqueuse de carbamate d'ammonium générée dans ladite unité de récupération d'urée (18) jusqu'audit second réacteur (28).
